# EUROPEAN PATENT APPLICATION

(11) **EP 3 943 094 A1**
(43) Date of publication of application: **26.01.2022**
(21) Application number: 19921788.6
(22) Date of filing: 17.07.2019
(51) Int. Cl.: A61K 35/744, A61K 35/747, A23L 33/135, A23K 10/16, A61P 27/12, A61P 25/00

(54) **COMPOSITION FOR INHIBITING NON-FLUORESCENT ADVANCED GLYCATION END PRODUCTS, AND USE THEREOF**

(30) Priority: 22.03.2019 KR 20190033130
(71) Applicant: Korea Food Research Institute, Wanju-gun, Jeollabuk-do 55365 (KR)
(72) Inventor: PARK, Ho Young, Jeonju-si Jeollabuk-do 54866 (KR); KIM, Yoon Sook, Seoul 04732 (KR); OH, Mi Jin, Jeonju-si Jeollabuk-do 54839 (KR); LEE, Sang Hoon, Jeonju-si Jeollabuk-do 54864 (KR); HA, Sang Keun, Suwon-si Gyeonggi-do 16700 (KR); CHOI, Sang Yoon, Seoul 06356 (KR); PARK, Yong Kon, Suwon-si Gyeonggi-do 16699 (KR)
(74) Representative: Snaith, James Michael
(86) International application number: PCT/KR2019/008836
(87) International publication number: WO 2020/196992

(57) **Abstract**

The present invention relates to a composition for inhibiting non-fluorescent advanced glycation end products, and a use thereof. More specifically, the present invention relates to: a composition for inhibiting non-fluorescent advanced glycation end products, comprising one or more strains selected from the group consisting of Lactobacillus sp. strains, Lactococcus sp. strains, Enterococcus sp. strains, Bifidobacterium sp. strains, Bacillus sp. strains, Saccharomyces sp. strains, Pediococcus sp. strains, and Leuconostoc sp. strains; and a food composition, a pharmaceutical composition, a composition for intestinal regulation, a probiotic composition, a feed composition, and a fermented product, which have an activity of reducing non-fluorescent advanced glycation end products and comprise the composition. In addition, the present invention relates to a method for producing a non-fluorescent advanced glycation end product inhibitor and a method for inhibiting non-fluorescent advanced glycation end products.

## Description

### Technical Field

The present invention relates to a composition for inhibiting non-fluorescent advanced glycation end products, a use thereof, and a method for inhibiting non-fluorescent advanced glycation end products using the same.

### Background Art

Nonenzymatic glycation reaction of proteins refers to Maillard reaction occurring between an amino acid group of a protein, such as lysine residue, and a reducing sugar without enzymatic action, which leads to the formation of advanced glycation end products (AGEs). Nonenzymatic glycation reaction of proteins is a reaction in which a free amino group of a protein, such as lysine or arginine, reacts with a carbonyl group of a reducing sugar to form an initial glycation reaction product, Schiff base, and the compounds formed therefrom continually produce a brown compound (melanoidine) through a series of complex reactions, such as condensation, rearrangement, oxidation, cleavage, cyclization, which leads to the formation of general concept of irreversible advanced glycation end products.

The advanced glycation end product is an irreversible reaction product. Therefore, even if blood glucose level is recovered normally, the product formed once is not degraded and accumulates in the tissues during the life of the protein, resulting in an abnormal change of the structure and function of the tissues. Glycation reaction easily occurs in a collagen with a relatively long half-life, and the advanced glycation end products formed once form a crosslinkage with a collagen to result in an abnormal physicochemical change of the structure in the body and other proteins of connective tissue and others. In addition, it is recognized by a specific receptor for various types of cells, which leads to the cause of diseases, such as diabetes, diabetes complications, such as diabetic retinopathy, diabetic neuropathy, diabetic cataract, diabetic nephropathy, chronic kidney disease, heart disease, vascular disease, and aging.

Therefore, studies have been in progress to inhibit the production of such advanced glycation end products. One of the representative synthetic pharmaceutical products is aminoguanidine, a nucleophilic hydrazine, which is bound with a product of condensation reaction to inhibit the production of advanced glycation end products, resulting in the prevention of the development of complications. In addition, aminoguanidine is the most promising pharmaceutical product for the prevention and treatment of diabetes complications, and thus, it has been studied in a phase 3 clinical trial. However, it was found that there was a problem of toxicity induced by prolonged administration of aminoguanidine, which calls a need for the development of safer pharmaceutical products.

Accordingly, there is a need for the development of a new ingredient which can inhibit and reduce advanced glycation end products and which secures no side effects for human body and stability.

In this regard, the present inventors have completed the present invention by developing an effective method for reducing advanced glycation end products using lactic acid bacteria.

### Detailed Description of the Invention

### Technical Problem

An object of the present invention is to provide a composition for inhibiting non-fluorescent advanced glycation end products, comprising one or more strains selected from the group consisting of Lactobacillus sp. strains, Lactococcus sp. strains, Enterococcus sp. strains, Bifidobacterium sp. strains, Bacillus sp. strains, Saccharomyces sp. strains, Pediococcus sp. strains, and Leuconostoc sp. strains.

Another object of the present invention is to provide a health functional food comprising the composition for inhibiting non-fluorescent advanced glycation end products using lactic acid bacteria of the the present invention.

Another object of the present invention is to provide a pharmaceutical composition for preventing or treating a disease related to non-fluorescent advanced glycation end products, comprising the composition for inhibiting non-fluorescent advanced glycation end products using lactic acid bacteria of the the present invention.

Another object of the present invention is to provide a feed composition comprising the composition for inhibiting non-fluorescent advanced glycation end products using lactic acid bacteria of the the present invention.

Another object of the present invention is to provide a method for producing a non-fluorescent advanced glycation end product inhibitor, comprising a step of pulverizing one or more strains selected from the group consisting of Lactobacillus sp. strains, Lactococcus sp. strains, Enterococcus sp. strains, Bifidobacterium sp. strains, Bacillus sp. strains, Saccharomyces sp. strains, Pediococcus sp. strains, and Leuconostoc sp. strains, a lysate thereof, or a culture thereof.

Furthermore, another object of the present invention is to provide a method for inhibiting non-fluorescent advanced glycation end products using one or more strains selected from the group consisting of Lactobacillus sp. strains, Lactococcus sp. strains, Enterococcus sp. strains, Bifidobacterium sp. strains, Bacillus sp. strains, Saccharomyces sp. strains, Pediococcus sp. strains, and Leuconostoc sp. strains.

### Solution to Problem

In order to achieve the above object of the present invention, the present invention provides a composition for inhibiting non-fluorescent advanced glycation end products, comprising one or more strains selected from the group consisting of Lactobacillus sp. strains, Lactococcus sp. strains, Enterococcus sp. strains, Bifidobacterium sp. strains, Bacillus sp. strains, Saccharomyces sp. strains, Pediococcus sp. strains, and Leuconostoc sp. strains.

In one embodiment of the present invention, the Lactobacillus sp. strain may be Lactobacillus plantarum, Lactobacillus gasseri, Lactobacillus cassei, Lactobacillus helveticus, Lactobacillus sakei, Lactobacillus paracassei, Lactobacillus crispatus, Lactobacillus lactis, Lactobacillus salivarius, Lactobacillus bulgaricus, Lactobacillus acidophilus, Lactobacillus johnsonii, Lactobacillus brevis, Lactobacillus paracassei subsp. tolerans, Lactobacillus brevis, Lactobacillus pentosus, Lactobacillus plantarum subsp. plantarum, Lactobacillus paraplantarum, Lactobacillus rhamnosus, Lactobacillus paracassei KF00816 (accession number KCCM11998P), or Lactobacillus pentosus KF8 (accession number KCCM11997P).

In one embodiment of the present invention, the Lactococcus sp. strain may be Lactococcus lactis, Lactococcus chungangensis, Lactococcus fujiensis, Lactococcus lactis subsp. cremoris, Lactococcus lactis subsp. lactis, Lactococcus raffinolactis, Lactococcus garvieae, or Lactococcus lactis KF140 (accession number KCCM11673P).

In one embodiment of the present invention, the Enterococcus sp. strain may be Enterococcus faecium or Enterococcus faecalis.

In one embodiment of the present invention, the Bifidobacterium sp. strain may be Bifidobacterium longum, Bifidobacterium breve, Bifidobacterium animalis sub. lactis, or Bifidobacterium bifidum.

In one embodiment of the present invention, the Bacillus sp. strain may be Bacillus amyloliquefaciens, Bacillus atrophaeus, Bacillus subtilis, Bacillus mojavensis, Bacillus natto, Bacillus polyfermenticus, or Bacillus subtilis KF11 (accession number KCCM11981P).

In one embodiment of the present invention, the Saccharomyces sp. strain may be Saccharomyces cerevisiae, Saccharomyces boulardii, Saccharomyces ellipsoideus, Saccharomyces pastorianus, or Saccharomyces bayanus.

In one embodiment of the present invention, the Pediococcus sp. strain may be Pediococcus pentosaceus or Pediococcus acidilactici.

In one embodiment of the present invention, the Leuconostoc sp. strain may be Leuconostoc citreum.

In one embodiment of the present invention, the non-fluorescent advanced glycation end product may be N-carboxymethyl lysine (CML) or N-carboxyethyl lysine (CEL).

In addition, the present invention provides a food composition comprising the composition for inhibiting non-fluorescent advanced glycation end products of the present invention.

In addition, the present invention provides a pharmaceutical composition for preventing or treating a disease related to non-fluorescent advanced glycation end products, comprising the composition for inhibiting non-fluorescent advanced glycation end products of the present invention.

In one embodiment of the present invention, the disease may be selected from the group consisting of diabetes, diabetes complications, chronic kidney disease, heart disease, vascular disease, and aging.

In addition, the present invention provides a feed composition comprising the composition for inhibiting non-fluorescent advanced glycation end products of the present invention.

In addition, the present invention provides a composition for intestinal regulation, comprising the composition for inhibiting non-fluorescent advanced glycation end products of the present invention.

In addition, the present invention provides a probiotic composition comprising the composition for inhibiting non-fluorescent advanced glycation end products of the present invention.

In addition, the present invention provides a fermented product comprising the composition for inhibiting non-fluorescent advanced glycation end products of the present invention.

In addition, the present invention provides a method for producing a non-fluorescent advanced glycation end product inhibitor, comprising a step of pulverizing a single strain or two or more mixed strains selected from the group consisting of Lactobacillus sp. strains, Lactococcus sp. strains, Enterococcus sp. strains, Bifidobacterium sp. strains, Bacillus sp. strains, Saccharomyces sp. strains, Pediococcus sp. strains, and Leuconostoc sp. strains, a lysate thereof, or a culture thereof.

In addition, the present invention provides a method for inhibiting non-fluorescent advanced glycation end products using a single strain or two or more mixed strains selected from the group consisting of Lactobacillus sp. strains, Lactococcus sp. strains, Enterococcus sp. strains, Bifidobacterium sp. strains, Bacillus sp. strains, Saccharomyces sp. strains, Pediococcus sp. strains, and Leuconostoc sp. strains.

### Effect of the Invention

The present invention relates to a composition for inhibiting non-fluorescent advanced glycation end products using lactic acid bacteria, and a use thereof. The composition for inhibiting non-fluorescent advanced glycation end products using lactic acid bacteria according to the present invention has an activity that can effectively reduce advanced glycation end products, and thus, has an effect that can be used for the manufacture of functional foods and pharmaceuticals that can prevent, improve or treat diseases caused by advanced glycation end products. Furthermore, it also has an effect that can be used for the manufacture of a composition for intestinal regulation, a probiotic composition, a feed composition, and a fermented product.

### Brief Description of Drawings

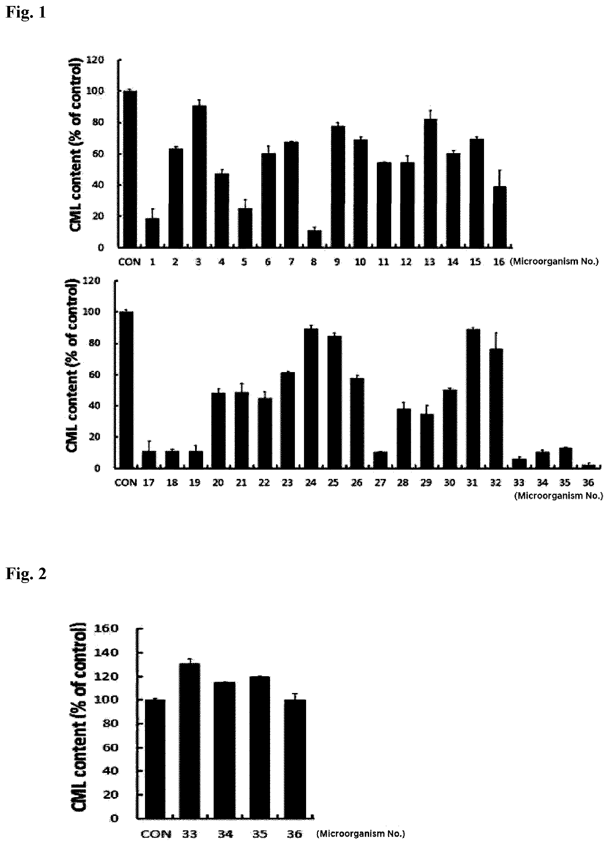

Fig. 1 shows the results of analyzing the degree of inhibition of advanced glycation end products in the case of microorganism treatment of the food containing advanced glycation end products (CML) in one embodiment of the present invention.
Fig. 2 shows the results of microorganisms without activity of inhibiting advanced glycation end products in the case of microorganism treatment of the food containing advanced glycation end products (CML) in one embodiment of the present invention.

### Best Mode for Carrying out the Invention

The present invention provides a composition for inhibiting non-fluorescent advanced glycation end products, and a use thereof. Specifically, the present invention provides a composition for inhibiting non-fluorescent advanced glycation end products using lactic acid bacteria, and a method for reducing non-fluorescent advanced glycation end products.

As previously described in the prior art, advanced glycation end products (AGEs) are irreversible reaction products. Therefore, even if blood glucose level is recovered normally, the products formed once are not degraded and accumulates in the tissues during the life of the protein to result in an abnormal change of the structure and function of the tissues, which leads to the cause of various diseases, such as diabetes, diabetes complications, chronic kidney disease, heart disease, vascular disease, or aging.

Types of advanced glycation end products include fluorescent substances, such as pentosidine or argpyrimidine, and non-fluorescent substances, such as N-carboxymethyl lysine (CML) and N-carboxyethyl lysine (CEL). The advanced glycation end products to be inhibited in the present invention are non-fluorescent advanced glycation end products.

With regard to the risk of CML among non-fluorescent advanced glycation end products, as a result of comparing the CML level of diabetic retinopathy patients and the CML level of normal people, it has been known that the expression level of CML is increased in diabetic retinopathy patients compared to normal people. In addition, it has been reported that diabetic retinopathy can be treated by inhibiting CML.

In addition, it has been known that the CML level of diabetic cataract patients is increased compared to the CML level of non-diabetic cataract patients. It has been found that the CML is a main cause of diabetic cataract. The CML level is increased when a diabetic nephropathy disease occurs. Thus, it has been known that the CML is a factor causing a diabetic nephropathy disease.

In addition, it has been found that the CML level is increased compared to the normal level, when chronic kidney disease occurs. Thus, it has been known that the increased CML level may be used as a factor indicating an occurrence of chronic kidney disease, and the CML inhibitor may be a therapeutic agent of chronic kidney disease.

In addition, it has been known that CML is a risk factor of diabetic neuropathy. In the research result, it has been reported that the CML level of diabetic neuropathy patients is remarkably increased as compared to the CML level of normal patients.

In the case of vascular disease, the CML level is also increased when vascular disease occurs, and thus, it has been known that CML is a risk factor of vascular disease, and it has been known that vascular disease can be prevented or treated by inhibiting CML. It has been known that CML is a main cause of heart disease and diabetes, and the CML level is increased when these diseases occur.

Therefore, when the production of such non-fluorescent advanced glycation end products is inhibited or reduced, various diseases caused by the advanced glycation end products can be prevented, improved, or treated.

Accordingly, the present inventors confirmed that, while researching an effective method for reducing non-fluorescent advanced glycation end products, the use of microorganisms effectively inhibited non-fluorescent advanced glycation end products.

Therefore, the present invention provides a composition for inhibiting non-fluorescent advanced glycation end products, comprising one or more strains selected from the group consisting of Lactobacillus sp. strains, Lactococcus sp. strains, Enterococcus sp. strains, Bifidobacterium sp. strains, Bacillus sp. strains, Saccharomyces sp. strains, Pediococcus sp. strains, and Leuconostoc sp. strains.

Here, the Lactobacillus sp. strain may be Lactobacillus plantarum, Lactobacillus gasseri, Lactobacillus cassei, Lactobacillus helveticus, Lactobacillus sakei, Lactobacillus paracassei, Lactobacillus crispatus, Lactobacillus lactis, Lactobacillus salivarius, Lactobacillus bulgaricus, Lactobacillus acidophilus, Lactobacillus johnsonii, Lactobacillus brevis, Lactobacillus paracassei subsp. tolerans, Lactobacillus brevis, Lactobacillus pentosus, Lactobacillus plantarum subsp. plantarum, Lactobacillus paraplantarum, Lactobacillus rhamnosus, Lactobacillus paracassei KF00816 (accession number KCCM11998P), or Lactobacillus pentosus KF8 (accession number KCCM11997P), but is not limited thereto.

The Lactococcus sp. strain may be Lactococcus lactis, Lactococcus chungangensis, Lactococcus fujiensis, Lactococcus lactis subsp. cremoris, Lactococcus lactis subsp. lactis, Lactococcus raffinolactis, Lactococcus garvieae, or Lactococcus lactis KF140 (accession number KCCM11673P), but is not limited thereto.

The Enterococcus sp. strain may be Enterococcus faecium or Enterococcus faecalis, but is not limited thereto.

The Bifidobacterium sp. strain may be Bifidobacterium longum, Bifidobacterium breve, Bifidobacterium animalis sub. lactis, or Bifidobacterium bifidum, but is not limited thereto.

The Bacillus sp. strain may be Bacillus amyloliquefaciens, Bacillus atrophaeus, Bacillus subtilis, Bacillus mojavensis, Bacillus natto, Bacillus polyfermenticus, or Bacillus subtilis KF11 (accession number KCCM11981P), but is not limited thereto.

The Saccharomyces sp. strain may be Saccharomyces cerevisiae, Saccharomyces boulardii, Saccharomyces ellipsoideus, Saccharomyces pastorianus, or Saccharomyces bayanus, but is not limited thereto.

The Pediococcus sp. strain may be Pediococcus pentosaceus or Pediococcus acidilactici, but is not limited thereto.

The Leuconostoc sp. strain may be Leuconostoc citreum.

In order to analyze an activity of reducing non-fluorescent advanced glycation end products for each microorganism alone and the mixed strain of the microorganisms described above, each strain was treated in a sample containing the advanced glycation end products, and then the content of the advanced glycation end products in the culture solution was measured. As a result, the present inventors confirmed that it showed an excellent effect of reducing the advanced glycation end products.

Therefore, the present inventors found that the above microorganisms, specifically, each microorganism alone or two or more mixed strains can be usefully used in the manufacture of a functional product for reducing non-fluorescent advanced glycation end products.

In one embodiment of the present invention, as a result of analyzing an activity of reducing CML, which is an advanced glycation end product, for each of 40 types of microorganisms, it was found that 36 microorganisms in the food containing CML had an activity of reducing CML, whereas 4 microorganisms did not have an activity of reducing CML, but rather CML was increased.

Therefore, the present invention can preferably provide a composition for inhibiting non-fluorescent advanced glycation end products, comprising one or more strains selected from the group consisting of 36 microorganisms described in Table 1 of the following Examples.

In addition, in another embodiment of the present invention, as a result of analyzing whether an activity of reducing CML in the use of mixed strains is better than that of the use of a single strain, it was confirmed that an activity of reducing CML was increased when two or more mixed strains of different microorganisms were used as compared to the single treatment group. The group treated with Lactobacillus paracassei KF00816 (accession number KCCM11998P), Lactobacillus pentosus KF8 (accession number KCCM11997P), Lactococcus lactis KF140 (accession number KCCM11673P) and Bacillus subtilis KF11 (accession number KCCM11981P) at a mixing ratio of 1:1:1:1 (the number of cells of microorganisms) was also shown to have an excellent activity of reducing CML. It was found that the group in which a total of 36 strains identified in the present invention were mixed had an activity of reducing CML to the extent that CML did not almost exist.

Therefore, the present invention can provide a composition for inhibiting advanced glycation end products, comprising the microorganism, a lysate thereof, or a culture thereof as an active ingredient.

In addition, the present invention can provide a health functional food composition comprising the composition for inhibiting non-fluorescent advanced glycation end products of the present invention.

The food composition of the present invention includes all forms of a functional food, a nutritional supplement, a health food, a food additive, and the like. The food composition of the above type may be manufactured in various forms according to the conventional methods known in the art. For example, as a health food, at least one of the group of the strains of the present invention itself, a lysate thereof, and a culture of the above strain may be drunken in the form of tea, juice, and drink, and may be ingested by granulation, encapsulation, and pulverization. In addition, at least one of the group of the strains of the present invention, a lysate thereof, and a culture thereof may be manufactured in the form of a composition by mixing with a known substance or an active ingredient known to have an effect of reducing advanced glycation end products. In addition, a functional food may be manufactured by adding at least one of the group of the strains of the present invention, a lysate thereof, and a culture thereof to beverages (including alcoholic beverages), fruits and their processed foods (for example, a canned fruit, a bottled fruit, jam, marmalade, etc.), fish, meat, and its processed food (for example, ham, sausage corned beef, etc.), breads and noodles (for example, udon, buckwheat noodle, ramen, spaghetti, macaroni, etc.), fruit juice, various drinks, cookies, taffy, dairy products (for example, butter, cheese, etc.), edible vegetable oil and fat, margarine, vegetable protein, retort food, frozen food, various seasonings (for example, bean paste, soy sauce, sauce, etc.), and the like.

The preferable content of the strain of the present invention, a lysate thereof, and a culture thereof, etc. in the food composition of the present invention is preferably, but not limited to, 0.01 to 50 wt% in a finally manufactured food. In addition, in order to use in the form of a food additive, at least one selected from the group consisting of the strains of the present invention, a lysate thereof, and a culture thereof as an active ingredient may be manufactured in the form of powder or concentrate.

In addition, the present invention may provide a pharmaceutical composition for preventing or treating a disease related to non-fluorescent advanced glycation end products, comprising the composition for inhibiting non-fluorescent advanced glycation end products of the present invention, and the disease may be diabetes, diabetes complications, chronic kidney disease, heart disease, vascular disease or aging, but is not limited thereto.

The pharmaceutical composition may contain a pharmaceutically acceptable salt alone or may further contain one or more pharmaceutically acceptable carriers, excipients, or diluents. The pharmaceutically acceptable carrier may further include, for example, a carrier for oral administration or a carrier for parenteral administration. Carriers for oral administration may include lactose, starch, cellulose derivatives, magnesium stearate, stearic acid, and the like. In addition, carriers for parenteral administration may include water, a suitable oil, saline, aqueous glucose, glycol, and the like, and may further include a stabilizer and a preservative. Suitable stabilizers include antioxidants such as sodium hydrogen sulfite, sodium sulfite or ascorbic acid. Suitable preservatives include benzalkonium chloride, methyl- or propyl-paraben and chlorobutanol. As other pharmaceutically acceptable carriers, reference may be made to those described in the literature (Remington's Pharmaceutical Sciences, 19th ed., Mack Publishing Company, Easton, PA, 1995).

The pharmaceutical composition of the present invention may be administered by any method to a mammal including a human. For example, it may be administered orally or parenterally. A parenteral administration method includes intravenous, intramuscular, intraarterial, intramedullary, intrathecal, intracardiac, transdermal, subcutaneous, intraperitoneal, intranasal, intestinal, topical, sublingual, or intracolonic administration, but is not limited thereto. Preferably, the pharmaceutical composition of the present invention may be administered orally or transdermally.

The pharmaceutical composition of the present invention may be formulated in a formulation for oral administration or a formulation for parenteral administration by the routes of administration as described above.

In the case of a formulation for oral administration, the composition of the present invention may be formulated using the methods known in the art into powder, granules, tablets, pills, dragees, capsules, liquids, gels, syrups, slurries, suspensions, and the like. For example, a formulation for oral administration may be obtained as a tablet or a dragee by blending an active ingredient with a solid excipient, milling them, adding a suitable adjuvant, and then, processing into a granule mixture. Examples of suitable excipients may include sugars, such as lactose, dextrose, sucrose, sorbitol, mannitol, xylitol, erythritol, maltitol, and the like, starches, such as corn starch, wheat starch, rice starch, potato starch, and the like, celluloses, such as cellulose, methyl cellulose, sodium carboxymethyl cellulose, hydroxypropyl methylcellulose, and the like, and fillers, such as gelatin, polyvinylpyrrolidone, etc. In addition, in some cases, cross-linked polyvinylpyrrolidone, agar, alginic acid, or sodium alginate, etc. may be added as a disintegrating agent. Furthermore, the pharmaceutical composition of the present invention may further comprise anticoagulants, lubricants, wetting agents, flavoring agents, emulsifiers, preservatives, and the like.

The formulation for parenteral administration may be formulated in the form of injections, creams, lotions, external ointments, oils, moisturizers, gels, aerosols, and nasal inhalers using the methods known in the art. These formulations are described in the literature (Remington's Pharmaceutical Science, 15th Edition, 1975. Mack Publishing Company, Easton, Pennsylvania 18042, Chapter 87: Blaug, Seymour), which is a formulary generally known in all pharmaceuticals and chemistry.

The total effective amount of the strain of the present invention itself, a lysate thereof, and a culture of the above strain, etc., may be administered to a patient as a single dose, and may be administered by fractionated treatment protocol, that is a long-term administration of multiple doses. The content of the active ingredient in the pharmaceutical composition of the present invention may vary depending on the severity of the disease. The preferable total dose of the strain of the present invention, a lysate thereof, and a culture thereof may be preferably about 0.01 ug to 1,000 mg, most preferably 0.1 ug to 100 mg, per 1 kg of patient's body weight per day. However, the dose of the strain of the present invention itself, a lysate thereof, and a culture of the above strain, etc., is determined upon consideration of routes of administration of the pharmaceutical composition and the number of times being treated, as well as various factors, such as patient's age, body weight, health condition, sex, severity of diseases, diet, excretion rate, and the like, for determining the effective dosage for the patient. Thus, upon consideration of such aspects, a person having ordinary skill in the art would be able to determine an appropriate effective dose according to the certain use of the strain of the present invention, a lysate thereof, and a culture thereof as an immune system enhancer. The pharmaceutical composition according to the present invention is not particularly limited to the formulations, routes of administration, and administration methods as long as the effect of the present invention shows.

In addition, the present invention provides a composition for intestinal regulation, a probiotic composition, a feed composition, comprising the composition for inhibiting non-fluorescent advanced glycation end products of the present invention. The composition for inhibiting non-fluorescent advanced glycation end products of the present invention may be used for the health enhancement of humans and animals, i.e., as a composition for intestinal regulation, a probiotic composition, or a feed composition. The above composition may comprise the above strain itself, a lysate thereof, and a culture of the above strain, etc., as an active ingredient, and may further comprise an excipient or a carrier. The above composition includes a culture solution itself cultured in a liquid medium, a filtrate (centrifuged supernatant), wherein a strain is removed by filtration or centrifugation of the above culture solution, etc. The content of the strain of the present invention in the composition may vary depending on the use and formulation of the composition.

The composition for intestinal regulation or the probiotic composition according to the present invention may be prepared and administered in various formulations and methods. For example, the strain used in the present invention, a lysate thereof, and a culture thereof may be prepared and administered in the form of tablets, troches, capsules, elixirs, syrups, powder, suspensions, granules, or the like by mixing with carriers and flavoring agents usually used in the pharmaceutical field.

Binders, glidants, disintegrating agents, excipients, solubilizers, dispersants, stabilizers, suspending agents, etc. may be used as a carrier. The mode of administration may use the oral, parenteral, or application method, and preferably, oral administration is preferred. In addition, the dosage may be appropriately selected, depending on the absorption rate, the inactivation rate, and the excretion rate of the active ingredient in the body, the age, sex, condition of a subject to be administered, etc. In addition, the feed composition according to the present invention may be prepared in the form of a fermented feed, a formula feed, a pellet form, a silage, etc.

The above fermented feed may be prepared by adding the composition for inhibiting non-fluorescent advanced glycation end products of the present invention and various microbial strains or enzymes to ferment the organic material, and the above formula feed may be prepared by mixing the strain of the present invention with various kinds of a regular feed. The feed in pellet form may be prepared by formulating the above fermented feed or the formula feed with a pelletizer, and the silage may be prepared by fermenting the green fodder with the strain according to the present invention.

In addition, the present invention provides a fermented product comprising the composition for inhibiting non-fluorescent advanced glycation end products of the present invention as an active ingredient. A method for producing the fermented product typically consists of the steps of the preparation of the raw material, the addition of lactic acid bacteria, fermentation, recovery of the finished product, etc. The step of preparing the raw material is a step of preparing a material to be fermented, and preparing the fermentation conditions such that the fermentation is well performed. The addition of lactic acid bacteria is the addition of a suitable amount of bacteria in accordance with the amount of a material to be fermented, wherein in the present invention, it is characterized in that the strain of the present invention is used. The fermentation is to be performed according to the fermentation conditions of conventional fermentation bacteria, wherein it is performed preferably at 37°C to 43°C for 4 to 168 hours. The recovery of the finished product includes removing the unnecessary byproducts or the unfermented materials from the ferments, as well as all customary work-up processes for facilitating storage and transportation, packaging, and the like. The fermented product prepared according to the method described above may be preferably a beverage, wherein the beverage refers to, for example, the product prepared by culturing the strain of the present invention to lactic acid fermentation, diluting it by adding sterilized water thereto, adding sugars, flavoring agents, etc., and then, filling a container therewith. Because the beverage generally contains lactic acid bacteria that are alive, the action of intestinal regulation in intestine after drinking can be expected.

Furthermore, the present invention provides a method for producing a non-fluorescent advanced glycation end product inhibitor, comprising a step of pulverizing a single strain or two or more mixed strains selected from the group consisting of Lactobacillus sp. strains, Lactococcus sp. strains, Enterococcus sp. strains, Bifidobacterium sp. strains, Bacillus sp. strains, Saccharomyces sp. strains, Pediococcus sp. strains, and Leuconostoc sp. strains, a lysate thereof, or a culture thereof.

In addition, the present invention provides a method for inhibiting non-fluorescent advanced glycation end products using one or more strains selected from the group consisting of Lactobacillus sp. strains, Lactococcus sp. strains, Enterococcus sp. strains, Bifidobacterium sp. strains, Bacillus sp. strains, Saccharomyces sp. strains, Pediococcus sp. strains, and Leuconostoc sp. strains.

Hereinafter, the present invention will be described in more detail through the examples. These Examples are for illustrating the present invention in more detail, and the scope of the present invention is not limited to these Examples.

### <Example 1>

### Screening of microorganisms having activity of reducing non-fluorescent advanced glycation end products

In order to develop a non-fluorescent advanced glycation end product inhibitor using microorganisms, microorganisms having an activity of reducing CML (Nε-(carboxymethyl)-L-lysine), a non-fluorescent advanced glycation end product, were first screened. For this, a food containing CML was prepared, and each microorganism was added to the food, and then the change in the content of CML was measured.

### <1-1> Preparation of CML-containing food

First, the CML-containing food was prepared. That is, casein, a milk protein, and lactose, a milk sugar, were used in order to reduce the CML produced during the process of heat treatment of milk. Sodium caseinate and D-lactose were purchased from ES FOOD and Sigma-Aldrich, respectively. Upon consideration of a ratio of casein to lactose in milk, a milk protein (casein) and a sugar (lactose) were mixed in a ratio of 1:7 and reacted at a temperature of 140°C for 80 minutes, which was used to evaluate the efficacy of reducing CML for the microorganisms of the present invention. At this time, it was confirmed that the CML content by Maillard reaction of casein and lactose was 5.90 ug/mL.

### <1-2> Analysis of CML content

Various types of microorganisms were added to the CML-containing food (solution) prepared in <1-1> above and cultured for 24 hours, and then the changed amount of N^{ε}-(carboxymethyl)lysine (CML) was measured with a CML ELISA kit (CircuLex, Ina, Nagano, Japan). The supernatant or standard sample and the anti-CML-adduct monoclonal antibody were added to 96 wells pre-coated with CML-BSA and reacted at room temperature for 1 hour. After washing four times with wash buffer, 100 µl of the HRP conjugated detection antibody was added, reacted for 1 hour, and then washed four times with wash buffer, and 100 µl of a substrate-containing solution was added and reacted under the conditions of light protection for 20 minutes, and then 100 µl of a stop solution was added, and the absorbance was measured at 450 nm using a microplate reader. The results are shown in the table below. In this case, the negative control group (CON; control) was a group containing CML in content of 5.90 ug/mL as measured by Maillard reaction of <1-1> above, in which microorganism was not added at all, and a CML content of the negative control group was set to 100% as a basis of analysis. Among the analyzed microorganisms, microorganisms with CML inhibitory activity are shown in Table 1 below, and microorganisms without CML inhibitory activity are shown in Table 2 below. In addition, microorganisms of No. 33, 34 and 35 in Table 1 below are strains newly discovered, identified and deposited by the present inventors through prior research. *Lactococcus lactis* KF140 is a strain that was deposited at the Korean Culture Center of Microorganisms (KCCM), which is an international depositary authority, and assigned with an accession number KCCM 11673P; Bacillus subtilis KF11 is a strain that was deposited on February 24, 2017 at the Korean Culture Center of Microorganisms (KCCM), which is an international depositary authority, and assigned with an accession number KCCM 11981P; a Lactobacillus pentosus KF8 strain is a strain that was deposited on March 24, 2017 at the Korean Culture Center of Microorganisms (KCCM), which is an international depositary authority, and assigned with an accession number KCCM 11997P; and Lactobacillus paracassei KF00816 is a strain that was deposited on March 24, 2017 at the Korean Culture Center of Microorganisms (KCCM), which is an international depositary authority, and assigned with an accession number KCCM 11998P.

**[Table 1] Microorganism with CML inhibitory activity**

| No. | Strain Name | Medium | Temperature | CML content (ug/ml) | CML content (%) |
|---|---|---|---|---|---|
| CON | No Microorganism | | | 5.9 | 100 |
| 1 | Lactobacillus plantarum | MRS | 37°C | 1.1 | 18.7 |
| 2 | Lactobacillus helveticus | MRS | 37°C | 3.73 | 63.2 |
| 3 | Lactobacillus cassei | MRS | 37°C | 5.38 | 91.1 |
| 4 | Lactobacillus paracassei | MRS | 37°C | 2.78 | 47.1 |
| 5 | Lactobacillus crispatus | MRS | 37°C | 1.47 | 24.9 |
| 6 | Lactobacillus sakei | MRS | 37°C | 3.57 | 60.5 |
| 7 | Lactobacillus lactis | MRS | 37°C | 3.99 | 67.6 |
| 8 | Lactobacillus salivarius | MRS | 37°C | 0.65 | 11.1 |
| 9 | Lactobacillus rhamnosus | MRS | 37°C | 4.6 | 77.9 |
| 10 | Lactobacillus bulgaricus | MRS | 42°C | 4.09 | 69.3 |
| 11 | Lactobacillus acidophilus | MRS | 37°C | 3.21 | 54.4 |
| 12 | Lactobacillus gasseri | MRS | 37°C | 3.21 | 54.4 |
| 13 | Lactobacillus j ohnsonii | MRS | 37°C | 4.86 | 82.3 |
| 14 | Lactobacillus helveticus | MRS | 37°C | 3.58 | 60.6 |
| 15 | Lactobacillus brevis | MRS | 37°C | 4.12 | 69.8 |
| 16 | Lactobacillus paracasei subsp. Tolerans | MRS | 37°C | 3.2 | 38.9 |
| 17 | Lactobacillus pentosus | MRS | 37°C | 0.65 | 11.1 |
| 18 | Lactobacillus plantarum subsp. Plantarum | MRS | 37°C | 0.66 | 11.1 |
| 19 | Lactobacillus paraplantarum | MRS | 37°C | 0.66 | 11.1 |
| 20 | Lactococcus lactis | MRS | 37°C | 2.84 | 48.1 |
| 21 | Enterococcus faecalis | MRS | 37°C | 2.87 | 48.6 |
| 22 | Enterococcus faecium | MRS | 37°C | 2.65 | 44.9 |
| 23 | Bifidobacterium longum | mMRS | 37°C | 3.63 | 61.5 |
| 24 | Bifidobacterium breve | mMRS | 37°C | 5.28 | 89.5 |
| 25 | Bifidobacterium animalis sub. Lactis | mMRS | 37°C | 4.99 | 84.6 |
| 26 | Bifidobacterium bifidum | mMRS | 37°C | 3.42 | 57.9 |
| 27 | Bacillus natto | MRS | 30°C | 0.62 | 10.5 |
| 28 | Bacillus subtilis | MRS | 30°C | 2.24 | 37.9 |
| 29 | Bacillus polyfermenticus | MRS | 30°C | 2.04 | 34.5 |
| 30 | Bacillus amyloliquefaciens | MRS | 30°C | 2.97 | 50.3 |
| 31 | Saccahromyces cerevisiae | MRS | 37°C | 5.26 | 89.2 |
| 32 | Pediococcus pentosaceus | MRS | 37°C | 4.5 | 76.2 |
| 33 | Lactococcus lactis KF140 (accession number KCCM11673P) | MRS | 37°C | 0.0009 | 6.2 |
| 34 | Bacillus subtilis KF11 (accession number KCCM11981P) | MRS | 37°C | 0.62 | 10.6 |
| 35 | Lactobacillus pentosus KF8 (accession number KCCM11997P) | MRS | 37°C | 0.81 | 13.6 |
| 36 | Lactobacillus paracasei KF00816 (accession number KCCM11998P) | MRS | 37°C | 1.19 | 20.2 |

**[Table 2] Microorganism without CML inhibitory activity**

| No. | Strain Name | Medium | temperature | CMLcontent (ug/ml) | CML content (%) |
|---|---|---|---|---|---|
| CON | No Microorganism | | | 5.9 | 100 |
| 37 | Lactobacillus reuteri | MRS | 37°C | 7.72 | 130.8 |
| 38 | Lactobacillus fermentum | MRS | 37°C | 6.77 | 114.7 |
| 39 | Lactobacillus oris | MRS | 37°C | 7.05 | 119.4 |
| 40 | Weissellacibaria | MRS | 37°C | 5.9 | 100 |

As a result of the analysis, as shown in Tables 1 and 2, it was found that all microorganisms, particularly all lactic acid bacteria, do not have an activity of inhibiting non-fluorescent advanced glycation end products. The present inventors found that the 36 microorganisms listed in Table 1 above have an activity of reducing CML.

Through these results, the present inventors found that when using the microorganisms in Table 1 above identified in the present invention, non-fluorescent advanced glycation end products such as CML could be effectively inhibited, and in particular, when treating the food containing advanced glycation end products, non-fluorescent advanced glycation end products in the food could be effectively inhibited.

As described above, the present invention has been described mainly based upon the preferable examples. A person having ordinary skill in the art to which the present invention belongs would be able to understand that the present invention may be implemented in a modified form within the scope which does not deviate from the essential characteristics of the present invention. Therefore, the examples disclosed above should be considered from an explanatory point of view, not a limited point of view. The scope of the present invention is defined by the claims, not the foregoing description, and all of the differences within the scope equivalent thereto should be interpreted to be included in the scope of the present invention.

The present patent is the result of a national research task project, (1) a task corresponding to task identification number E0164400-04, ministry name: Ministry of Science and ICT, research management specialized agency: Korea Food Research Institute, managing department: Korea Food Research Institute, research project name: a project with the unique role of the institute, research task title: Development of food and pharmaceutical materials for reducing and improving risk factors for diabetes complications, research period: January 1, 2019 to December 31, 2019; and (2) a task corresponding to task identification number E0164400-03, ministry name: Ministry of Science and ICT, research management specialized agency: Korea Food Research Institute, managing department: Korea Food Research Institute, research project name: a project with the unique role of the institute, research task title: Development of technology for analyzing and reducing major causative factors for diabetes complications, research period: January 1, 2018 to December 31, 2018.

### [Accession number]

Depositary authority name: Korean Culture Center of Microorganisms (foreign country) Accession number: KCCM11997P
Deposit date: March 24, 2017
Depositary authority name: Korean Culture Center of Microorganisms (foreign country) Accession number: KCCM11998P
Deposit date: March 24, 2017
Depositary authority name: Korean Culture Center of Microorganisms (foreign country) Accession number: KCCM11673P
Deposit date: March 6, 2015
Depositary authority name: Korean Culture Center of Microorganisms (foreign country) Accession number: KCCM11981P
Deposit date: February 24, 2017

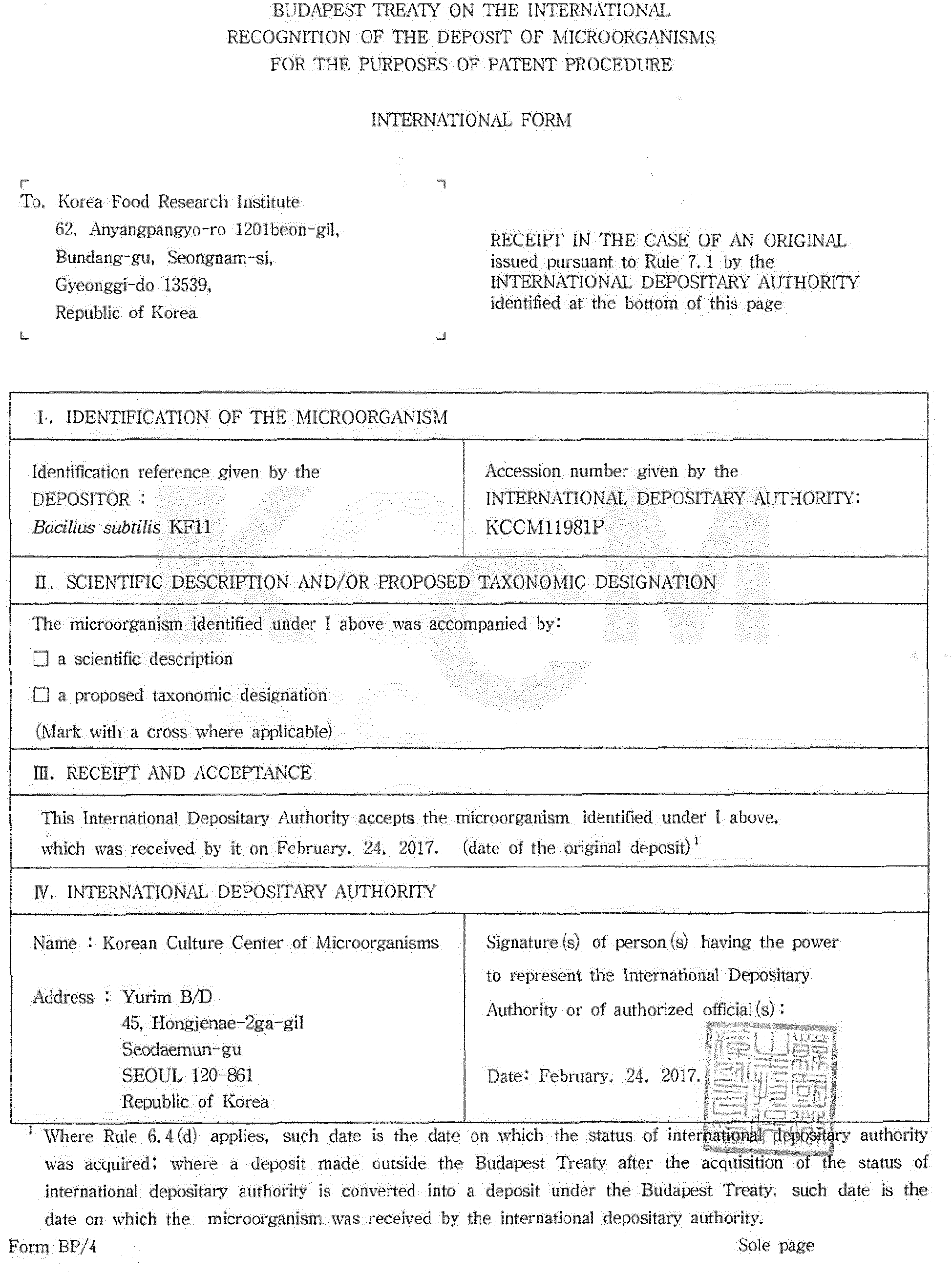

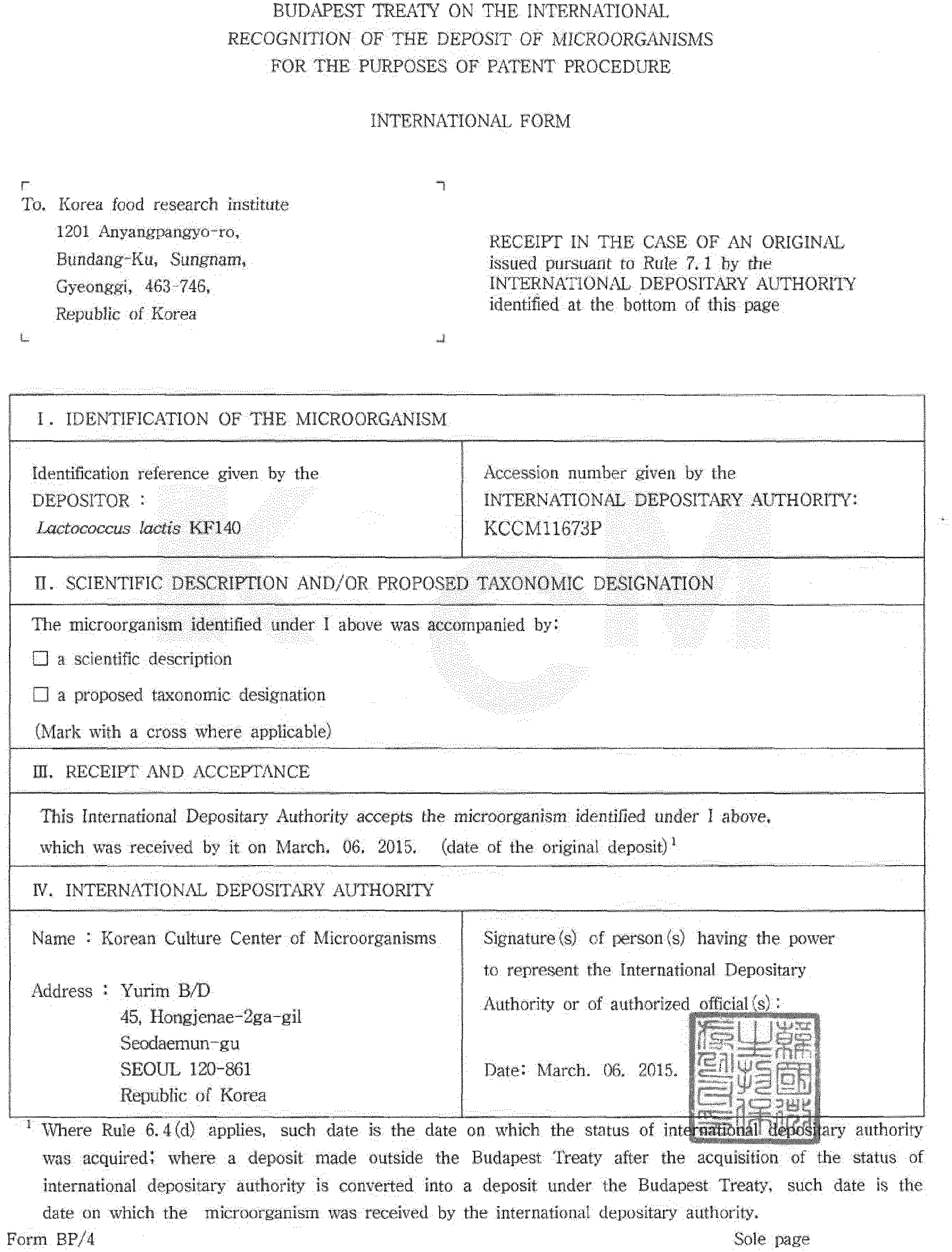

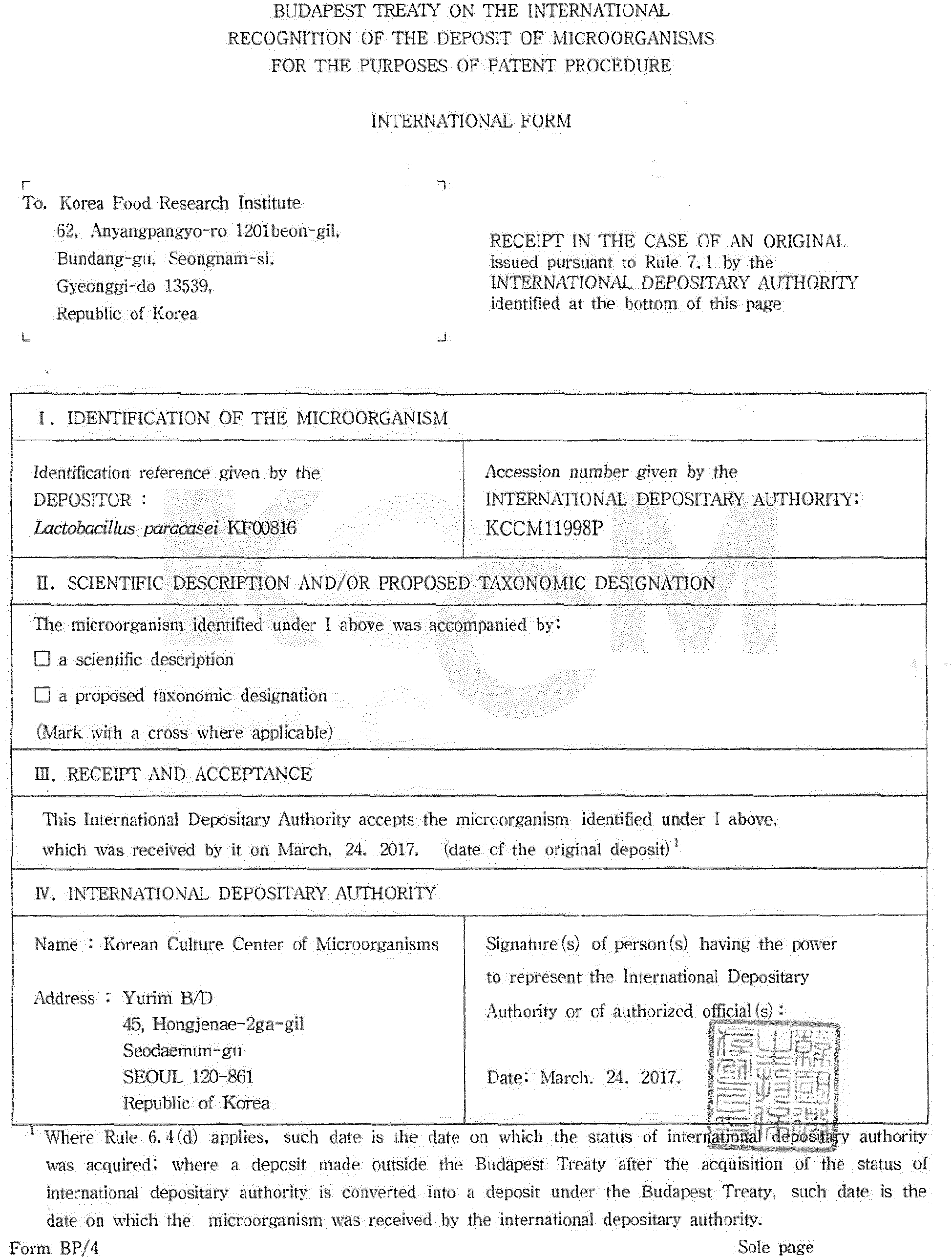

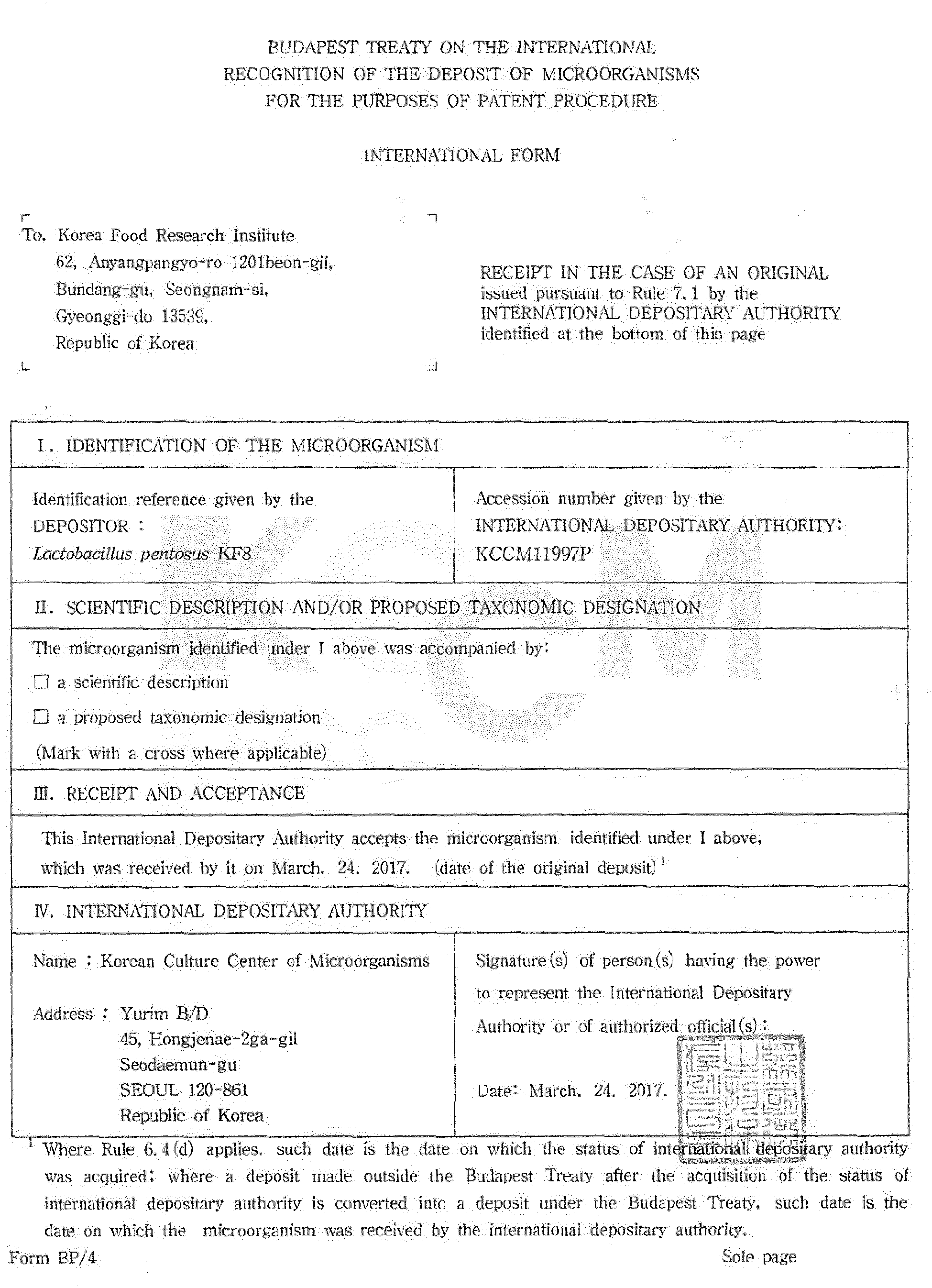

## Claims

1. A composition for inhibiting non-fluorescent advanced glycation end products, comprising one or more strains selected from the group consisting of Lactobacillus sp. strains, Lactococcus sp. strains, Enterococcus sp. strains, Bifidobacterium sp. strains, Bacillus sp. strains, Saccharomyces sp. strains, Pediococcus sp. strains, and Leuconostoc sp. strains.

2. The composition for inhibiting non-fluorescent advanced glycation end products according to claim 1, **characterized in that** the Lactobacillus sp. strain is selected from the group consisting of Lactobacillus plantarum, Lactobacillus gasseri, Lactobacillus cassei, Lactobacillus helveticus, Lactobacillus sakei, Lactobacillus paracassei, Lactobacillus crispatus, Lactobacillus lactis, Lactobacillus salivarius, Lactobacillus bulgaricus, Lactobacillus acidophilus, Lactobacillus johnsonii, Lactobacillus brevis, Lactobacillus paracassei subsp. tolerans, Lactobacillus brevis, Lactobacillus pentosus, Lactobacillus plantarum subsp. plantarum, Lactobacillus paraplantarum, Lactobacillus rhamnosus, Lactobacillus paracassei KF00816 (accession number KCCM11998P), and Lactobacillus pentosus KF8 (accession number KCCM11997P).

3. The composition for inhibiting non-fluorescent advanced glycation end products according to claim 1, **characterized in that** the Lactococcus sp. strain is selected from the group consisting of Lactococcus lactis, Lactococcus chungangensis, Lactococcus fujiensis, Lactococcus lactis subsp. cremoris, Lactococcus lactis subsp. lactis, Lactococcus raffinolactis, Lactococcus garvieae, and Lactococcus lactis KF140 (accession number KCCM11673P).

4. The composition for inhibiting non-fluorescent advanced glycation end products according to claim 1, **characterized in that** the Enterococcus sp. strain is Enterococcus faecium or Enterococcus faecalis.

5. The composition for inhibiting non-fluorescent advanced glycation end products according to claim 1, **characterized in that** the Bifidobacterium sp. strain is Bifidobacterium longum, Bifidobacterium breve, Bifidobacterium animalis sub. lactis, or Bifidobacterium bifidum.

6. The composition for inhibiting non-fluorescent advanced glycation end products according to claim 1, **characterized in that** the Bacillus sp. strain is Bacillus amyloliquefaciens, Bacillus atrophaeus, Bacillus subtilis, Bacillus mojavensis, Bacillus natto, Bacillus polyfermenticus, or Bacillus subtilis KF11 (accession number KCCM11981P).

7. The composition for inhibiting non-fluorescent advanced glycation end products according to claim 1, **characterized in that** the Saccharomyces sp. strain is Saccharomyces cerevisiae, Saccharomyces boulardii, Saccharomyces ellipsoideus, Saccharomyces pastorianus, or Saccharomyces bayanus.

8. The composition for inhibiting non-fluorescent advanced glycation end products according to claim 1, **characterized in that** the Pediococcus sp. strain is Pediococcus pentosaceus or Pediococcus acidilactici.

9. The composition for inhibiting non-fluorescent advanced glycation end products according to claim 1, **characterized in that** the Leuconostoc sp. strain is Leuconostoc citreum.

10. The composition for inhibiting non-fluorescent advanced glycation end products according to claim 1, **characterized in that** the non-fluorescent advanced glycation end product is N-carboxymethyl lysine (CML) or N-carboxyethyl lysine (CEL).

11. A food composition comprising the composition for inhibiting non-fluorescent advanced glycation end products of claim 1.

12. A pharmaceutical composition for preventing or treating a disease related to non-fluorescent advanced glycation end products, comprising the composition for inhibiting non-fluorescent advanced glycation end products of claim 1.

13. The pharmaceutical composition for preventing or treating a disease related to non-fluorescent advanced glycation end products according to claim 12, **characterized in that** the disease is selected from the group consisting of diabetes, diabetes complications, chronic kidney disease, heart disease, vascular disease, and aging.

14. A feed composition comprising the composition for inhibiting non-fluorescent advanced glycation end products of claim 1.

15. A composition for intestinal regulation, comprising the composition for inhibiting non-fluorescent advanced glycation end products of claim 1.

16. A probiotic composition comprising the composition for inhibiting non-fluorescent advanced glycation end products of claim 1.

17. A fermented product comprising the composition for inhibiting non-fluorescent advanced glycation end products of claim 1.

18. A method for producing a non-fluorescent advanced glycation end product inhibitor, comprising a step of pulverizing a single strain or two or more mixed strains selected from the group consisting of Lactobacillus sp. strains, Lactococcus sp. strains, Enterococcus sp. strains, Bifidobacterium sp. strains, Bacillus sp. strains, Saccharomyces sp. strains, Pediococcus sp. strains, and Leuconostoc sp. strains, a lysate thereof, or a culture thereof.

19. A method for inhibiting non-fluorescent advanced glycation end products using a single strain or two or more mixed strains selected from the group consisting of Lactobacillus sp. strains, Lactococcus sp. strains, Enterococcus sp. strains, Bifidobacterium sp. strains, Bacillus sp. strains, Saccharomyces sp. strains, Pediococcus sp. strains, and Leuconostoc sp. strains.
